# EUROPEAN PATENT APPLICATION

(11) **EP 4 801 233 A1**
(43) Date of publication of application: **02.09.2026**
(21) Application number: 24882481.5
(22) Date of filing: 25.10.2024
(51) Int. Cl.: H10K 30/50, C07D 487/22, H10K 30/40, H10K 30/81, H10K 30/86, H10K 50/14, H10K 85/10, H10K 85/30, H10K 85/50

(54) **PHOTOELECTRIC CONVERSION ELEMENT, PHOTOELECTRIC CONVERSION DEVICE, MOBILE BODY, AND BUILDING MATERIAL**

(30) Priority: 27.10.2023 JP 2023184761; 27.10.2023 JP 2023184756; 27.10.2023 JP 2023184750; 21.12.2023 JP 2023216294; 21.12.2023 JP 2023216296; 21.12.2023 JP 2023216299; 16.02.2024 JP 2024022244; 16.02.2024 JP 2024022251; 16.02.2024 JP 2024022246; 10.05.2024 JP 2024077353; 23.10.2024 JP 2024186698
(71) Applicant: Canon Kabushiki Kaisha, Tokyo 146-8501 (JP)
(72) Inventor: SATO, Mizuki, Tokyo 146-8501 (JP); NISHIDA, Tsutomu, Tokyo 146-8501 (JP); NAKAMURA, Nobuhiro, Tokyo 146-8501 (JP); OHSAWA, Tatsuya, Tokyo 146-8501 (JP)
(74) Representative: TBK
(86) International application number: PCT/JP2024/038058
(87) International publication number: WO 2025/089373

(57) **Abstract**

To provide a photoelectric conversion element having improved durability and photoelectric conversion efficiency, the present invention provides a photoelectric conversion element including: a first electrode; a second electrode; and a photoelectric conversion layer arranged between the first electrode and the second electrode, the photoelectric conversion layer containing a crystal having a perovskite structure, wherein the photoelectric conversion element includes a charge-transporting layer between the photoelectric conversion layer and the first electrode, wherein the charge-transporting layer contains a P-type semiconductor crystal and a resin, and wherein the P-type semiconductor crystal has at least one functional group selected from the group consisting of: a hydroxy group; a carboxy group; an amino group; an imino group; and a sulfo group.

## Description

### [Technical Field]

The present invention relates to a photoelectric conversion element, a photoelectric conversion apparatus, a moving body, and a building material.

### [Background Art]

In order to solve a problem of the depletion of fossil energy and a global environmental problem caused by the use of the fossil energy, investigations on a renewable and clean alternative energy source, such as solar energy, wind power, or water power, have been actively performed. In particular, an interest in a solar cell that directly changes sunlight into electrical energy has been increasing. The term "solar cell" as used herein means a battery that generates a current-voltage through utilization of a photovoltaic effect in which light energy is absorbed from sunlight to generate an electron and a hole.

Recently, an n-p diode-type silicon (Si) single crystal-based solar cell having a light energy conversion efficiency of more than 20% has been widely known, and has been actually used in solar power generation. However, the solar cell requires a high temperature treatment step and the price of a material itself is high, and hence there is a problem in that the cost per unit electric power is high. In addition, there is a problem with its supply property in terms of a silicon resource.

Meanwhile, a solar cell using an organic material (hereinafter also referred to as "organic solar cell") does not require the high temperature treatment step, and can be produced in a so-called roll-to-roll system using a sheet-shaped substrate. Accordingly, cost reduction is expected. However, further improvements in power generation efficiency and durability have been desired for practical use of the organic solar cell. In particular, the development of a perovskite solar cell including a crystal having a perovskite structure as a photoelectric conversion layer toward its practical use has been advanced because the cell is excellent in photoelectric conversion characteristic.

For example, in Patent Literature 1, there is a description of a technology including incorporating an organic semiconductor and a polymer compound having a glass transition temperature of 100°C or more into a hole-transporting layer to alleviate its peeling from an electrode. In Non Patent Literature 1, there is a description that conversion efficiency is improved by mixing copper phthalocyanine and a conductive polymer into a hole-transporting layer.

### [Citation List]

### [Patent Literature]

PTL 1: Japanese Patent Laid-Open No. 2018-170382

### [Non Patent Literature]

NPL 1: Q. Hu, et al, Sol. RRL, 2019, 3, 1800264

### [Summary of Invention]

### [Technical Problem]

According to investigations made by the inventors of the present invention, it has been found that there is room for improvements in durability and photoelectric conversion efficiency of the photoelectric conversion elements described in Patent Literature 1 and Non Patent Literature 1.

Accordingly, the present invention is directed to providing a photoelectric conversion element having improved durability and photoelectric conversion efficiency. The present invention is also directed to providing a photoelectric conversion apparatus.

### [Solution to Problem]

The present invention is directed to a photoelectric conversion element including:
a first electrode; a second electrode; and a photoelectric conversion layer arranged between the first electrode and the second electrode, the photoelectric conversion layer containing a crystal having a perovskite structure,
wherein the photoelectric conversion element includes a charge-transporting layer between the photoelectric conversion layer and the first electrode,
wherein the charge-transporting layer contains a P-type semiconductor crystal and a resin, and
wherein the P-type semiconductor crystal has at least one functional group selected from the group consisting of: a hydroxy group; a carboxy group; an amino group; an imino group; and a sulfo group.

The present invention is also directed to a photoelectric conversion element including:
a first electrode; a second electrode; and a photoelectric conversion layer arranged between the first electrode and the second electrode, the photoelectric conversion layer containing a crystal having a perovskite structure,
wherein the photoelectric conversion element includes a charge-transporting layer between the photoelectric conversion layer and the first electrode,
wherein the charge-transporting layer contains a P-type semiconductor crystal and a resin, and
wherein the P-type semiconductor crystal has a functional group that may serve as a hydrogen bond donor.

The present invention is also directed to a photoelectric conversion element including:
a first electrode; a second electrode; and a photoelectric conversion layer arranged between the first electrode and the second electrode, the photoelectric conversion layer containing a crystal having a perovskite structure,
wherein the photoelectric conversion element includes a charge-transporting layer between the photoelectric conversion layer and the first electrode,
wherein the charge-transporting layer contains a P-type semiconductor crystal and a resin, and
wherein the P-type semiconductor crystal has a Lewis basic functional group.

The present invention is also directed to a photoelectric conversion apparatus including the above-mentioned photoelectric conversion element.

The present invention is also directed to a moving body including the above-mentioned photoelectric conversion element.

The present invention is also directed to a building material including the above-mentioned photoelectric conversion element.

### [Advantageous Effects of Invention]

According to the present invention, the photoelectric conversion element having improved durability and photoelectric conversion efficiency can be provided.

### [Brief Description of Drawings]

[Fig. 1]

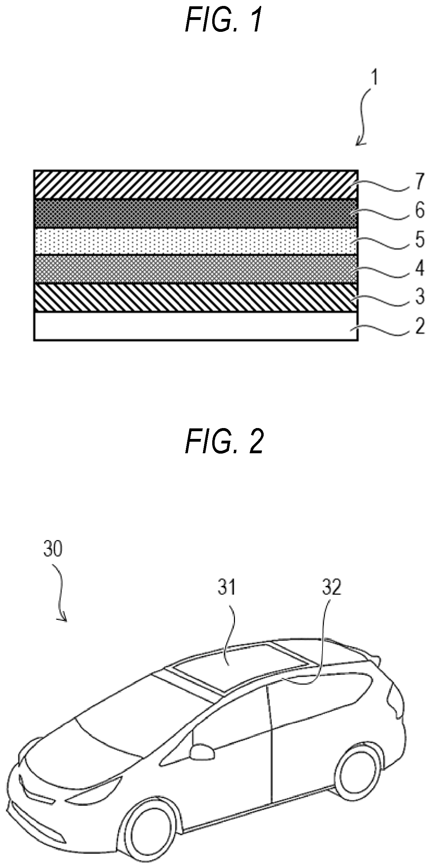

   Fig. 1 is a schematic diagram of the layer configuration in a thickness direction of a photoelectric conversion element according to one embodiment of the present invention.
[Fig. 2]
   Fig. 2 is a perspective view for schematically illustrating a moving body including the photoelectric conversion element according to one embodiment of the present invention.
[Fig. 3]

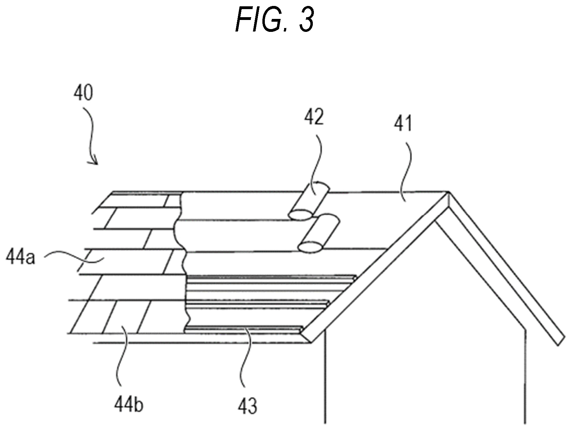

   Fig. 3 is a perspective view for schematically illustrating a building material including the photoelectric conversion element according to one embodiment of the present invention.

### [Description of Embodiments]

### <One Embodiment>

One embodiment is directed to a photoelectric conversion element.

A photoelectric conversion element of the present invention includes a first electrode, a second electrode, and a photoelectric conversion layer arranged between the first electrode and the second electrode, the photoelectric conversion layer containing a crystal having a perovskite structure, and is characterized in that the photoelectric conversion element includes a charge-transporting layer between the photoelectric conversion layer and the first electrode, the charge-transporting layer contains a P-type semiconductor crystal and a resin, and the P-type semiconductor crystal has at least one functional group selected from the group consisting of: a hydroxy group; a carboxy group; an amino group; an imino group; and a sulfo group.

In addition, a photoelectric conversion element of the present invention includes a first electrode, a second electrode, and a photoelectric conversion layer arranged between the first electrode and the second electrode, the photoelectric conversion layer containing a crystal having a perovskite structure, and is characterized in that the photoelectric conversion element includes a charge-transporting layer between the photoelectric conversion layer and the first electrode, the charge-transporting layer contains a P-type semiconductor crystal and a resin, and the P-type semiconductor crystal has a functional group that may serve as a hydrogen bond donor.

In addition, a photoelectric conversion element of the present invention includes a first electrode, a second electrode, and a photoelectric conversion layer arranged between the first electrode and the second electrode, the photoelectric conversion layer containing a crystal having a perovskite structure, and is characterized in that the photoelectric conversion element includes a charge-transporting layer between the photoelectric conversion layer and the first electrode, the charge-transporting layer contains a P-type semiconductor crystal and a resin, and the P-type semiconductor crystal has a Lewis basic functional group.

As a result of investigations, the inventors have found that a photoelectric conversion element having excellent durability and conversion efficiency is provided by including the above-mentioned charge-transporting layer. The reason is presumed as described below.

Several kinds of defects, such as under-coordinated Pb²⁺ ions, Pb clusters, iodine vacancies, organic A cation vacancies, and under-coordinated I⁻ ions, are included on a perovskite surface. Those surface defects may cause recombination of electrons and holes via non-radiative channels, and the recombination may lead to degradation of the photoelectric conversion element or a reduction in conversion efficiency thereof. In addition, in a perovskite solar cell, a phenomenon in which water molecules from the outside, or ions, molecules, and the like for forming the photoelectric conversion element migrate to another layer to destroy the structure of the photoelectric conversion element may occur.

In contrast, it is presumed that when a P-type semiconductor crystal having a Lewis basic functional group or a functional group that may serve as a hydrogen bond donor is included in the charge-transporting layer in contact with the photoelectric conversion layer, reductions in durability and conversion efficiency of the photoelectric conversion element can be prevented. Factors allowing this prevention are, for example, as follows: defects of a Lewis acid that are present on the perovskite surface are passivated (passivation) through interaction with a Lewis base; and under-coordinated I⁻ ions are trapped by hydrogen atoms included in the functional groups. Further, defects in a crystal having a perovskite structure extend from several tens to several hundreds of nanometers. Accordingly, it has been found that even when several tens of nanometers of a hole-transporting layer or an insulating layer is introduced between the charge-transporting layer and photoelectric conversion layer of the present invention, the inclusion of such charge-transporting layer contributes to improvements in durability and conversion efficiency of the photoelectric conversion element.

In addition, it is presumed that when the P-type semiconductor crystal and the resin are included in the charge-transporting layer, the defects and the like on the perovskite surface, which cannot be covered by the P-type semiconductor crystal alone, and the functional groups of the resin interact with each other. Further, the P-type semiconductor crystal, which has a high charge-transporting property and a high hole mobility, does not hinder charge transportation even when the crystal is included in a thick film. However, when the resin is further included in the charge-transporting layer, the photoelectric conversion layer can be reliably covered, and durability can be enhanced while charge transportation is maintained.

The effects of the present invention can be achieved when the respective configurations synergistically exhibit effects on each other through the mechanism described above.

The present invention is described in detail below by way of preferred embodiments. The present invention is not limited to the following embodiments, and the following embodiments, which are appropriately changed, modified, and the like based on the ordinary knowledge of a person skilled in the art without departing from the gist of the present invention, are also encompassed within the scope of the present invention.

The term "layer" as used herein means not only a layer having a clear boundary or a layer having a flat thin film shape but also a layer having a concentration gradient in which the concentration of a constituent element gradually changes, or a layer that may form a complicatedly intricate structure together with another layer. In addition, the elemental analysis of the layer may be performed by, for example, performing the TOF-SIMS/FE-TEM/EDS line analysis measurement of a cross section of the photoelectric conversion element and determining the element distribution of a specific element. The analysis of each layer may be performed by peeling and removing a layer from a completed element to expose the layer to be analyzed. For the quantification of a volume ratio, the area ratio of an exposed surface or a cross section may be used as the volume ratio of the layer.

Fig. 1 is a sectional view for schematically illustrating the configuration of the photoelectric conversion element according to one embodiment of the present invention. A photoelectric conversion element 1 includes a substrate 2, and a second electrode 3, an electron-transporting layer 4, a photoelectric conversion layer 5, a charge-transporting layer 6, and a first electrode 7 arranged thereon. One of the first electrode 7 and the second electrode 3 is an anode, and the other is a cathode. A current can be extracted by connecting the first electrode 7 and the second electrode 3 to an external circuit.

The photoelectric conversion layer 5 is excited by light that has entered the layer through the substrate 2, the second electrode 3, and the electron-transporting layer 4, or the first electrode 7 and the charge-transporting layer 6 to generate an electron or a hole. That is, the photoelectric conversion layer 5 generates a current between the first electrode 7 and the second electrode 3. The electron-transporting layer 4 is a layer arranged between the photoelectric conversion layer 5, and the two electrodes (the second electrode 3 and the first electrode 7), and may not be formed in some cases. A form in which the plurality of electron-transporting layers 4 and photoelectric conversion layers 5 are laminated may be adopted. Such form may also be referred to as "tandem structure." The respective members are described below. In addition, the photoelectric conversion element may be produced in the order of the first electrode 7, the charge-transporting layer 6, the photoelectric conversion layer 5, the electron-transporting layer 4, and the second electrode 3 on the substrate 2.

### [Photoelectric Conversion Element]

The photoelectric conversion element of the present invention is characterized by including: the first electrode; the second electrode; and the photoelectric conversion layer arranged between the first electrode and the second electrode, the photoelectric conversion layer containing the crystal having a perovskite structure. In addition, in order to improve the photoelectric conversion efficiency, a tandem type in which the photoelectric conversion elements are laminated may be adopted. The kind of the photoelectric conversion element to be laminated is not limited, and for example, a silicon solar cell or a CIGS solar cell may be adopted in addition to a perovskite solar cell using a perovskite crystal in its photoelectric conversion layer.

A method of forming each of the layers of the photoelectric conversion element of the present invention is, for example, a coating method or a vapor deposition method. Examples of the coating method include dip coating, spin coating, spray coating, ink jet coating, meniscus coating, screen coating, roll coating, die coating, blade coating, curtain coating, and wire bar coating. The coating method is a method including preparing a coating liquid for each layer to be described later, applying the liquid in the desired order of layers, and drying the liquid. A desired method may be selected as such forming method in accordance with each layer.

The respective layers are described below.

### [Substrate]

The photoelectric conversion element 1 of the present invention may include the substrate 2, and examples thereof include a transparent glass substrate made of soda-lime glass or alkali-free glass, a ceramic substrate, and a transparent plastic substrate. When light is taken in from the first electrode 7 side, an opaque material may be used as the substrate 2, and when light is taken in from the second electrode 3 side, the substrate 2 is formed of a transparent material.

### [Electrode]

A material for the first electrode 7 or the second electrode 3 is not particularly limited, and a material that has hitherto been known may be used. Examples thereof include: metals, such as gold, silver, titanium, and copper; sodium; a sodium-potassium alloy; lithium; magnesium; carbon; aluminum; a magnesium-silver mixture; a magnesium-indium mixture; an aluminum-lithium alloy; an Al/Al₂O₃ mixture; and an Al/LiF mixture.

Examples of a transparent electrode material include: conductive transparent materials, such as CuI, indium tin oxide (ITO), SnO₂, aluminum zinc oxide (AZO), indium zinc oxide (IZO), gallium zinc oxide (GZO), fluorine-doped tin oxide (FTO), and antimony-doped tin oxide (ATO); and conductive transparent polymers.

Those materials may be used alone or in combination thereof. At least one electrode of the first electrode 7 or the second electrode 3 on a light incident side is a transparent electrode, and the other may be a transparent electrode or may also serve as a reflective layer formed of a light reflective material, or may be a transparent electrode including a reflective layer on a side opposite to the light incident side. When the first electrode 7 is on the light incident side, the second electrode 3 and the substrate 2 may be a transparent electrode and a reflective layer, respectively. The electrode may be a patterned electrode.

### [Photoelectric Conversion Layer]

The photoelectric conversion layer 5 contains the crystal having a perovskite structure. The crystal having a perovskite structure to be used in the present invention is preferably represented by the following general formula [1].

ABX₃ [1]

In the general formula [1], A represents a monovalent cation of an organic molecule or a metal atom, B represents a divalent metal cation, and X represents a monovalent halide anion.

A in the general formula [1] preferably represents CₚN_{q}Hᵣ ("p", "q", and "r" each represent a positive integer) in the case of, for example, the organic molecule. Specific examples thereof include methylammonium and formamidium.

In addition, the metal atom is not particularly limited, and lithium, cesium, sodium, potassium, and rubidium are preferred. Those organic molecules or metal atoms may be used alone or in combination thereof.

When the cation A to be included is too large to fit in a crystal having a three-dimensional perovskite structure, a crystal having a two-dimensional perovskite structure, a crystal having a 2.5-dimensional perovskite structure with properties of both the two-dimensional and three-dimensional perovskite structures, a two-layer crystal having three-dimensional and two-dimensional perovskite structures, or a crystal having a mixed three-dimensional/two-dimensional perovskite structure is formed, and any of the structures functions as the photoelectric conversion layer.

The two-layer crystal having three-dimensional and two-dimensional perovskite structures refers to a crystal in which the crystals having three-dimensional and two-dimensional perovskite structures are laminated as independent and separate layers. The crystal having a mixed three-dimensional/two-dimensional perovskite structure refers to a crystal having a structure in which both the regions or domains of crystals having two-dimensional or 2.5-dimensional layered and three-dimensional perovskite structures are mixed.

It is preferred that the crystal having a two-dimensional perovskite or 2.5-dimensional perovskite structure be represented by each of the following general formulae [2] to [4] ("n" represents a positive integer).

R'₂Aₙ₋₁BₙX₃ₙ₊₁ [2]

R"Aₙ₋₁BₙX₃ₙ₊₁ [3]

R‴AₙBₙX₃ₙ₊₁ [4]

The general formula [2], the general formula [3], and the general formula [4] form perovskite structures of a Ruddlesden-Popper (RP) type, a Dion-Jacobson (DJ) type, and an Alternating cations in the interlayer (ACI) type, respectively.

R', R", and R‴ in the general formulae [2] to [4] each represent a cation of an organic molecule or a metal that may have a substituent. Specifically, ethylammonium, propylammonium, n-butylammonium, n-hexylammonium, n-octylammonium, 1,6-hexadiammonium, iso-butylammonium, 3-(nonafluoro-tert-butyloxy)propylamine, 1,3-propanediammonium, 1,5-pentamethylenediamine, octyldiammonium, 2,2-(ethylenedioxy)bis(ethylammonium), 5-aminovaleric acid, 4-tert-butylammonium, N,N'-dimethylethylene-1,2-diammonium, 2,2,3,3,3-pentafluoropropylammonium, guanidinium, propylammonium, propargylamine, alkylammonium, cyclohexylmethylammonium, 4-(aminomethyl)piperidinium, piperidinium, pyrrolidinium, cyclohexylammonium, 4-fluorophenethylammonium, 4-fluorophenethylammonium, trifluoromethylbenzylammonium, pentafluorobenzylammonium, pentafluorophenylethylammonium, 4-methoxyphenethylammonium, imidazolium, pyridinium, 3-thiophenemethylammonium, 2-thiopheneethylammonium, 2-thiopheneformamidium, 2-thiophenemethylammonium, 1-naphthylmethylammonium, 2-naphthylmethylammonium, phenethylammonium, phenylammonium, benzylammonium, 2,5-thiophenedimethylammonium, phenylpropylammonium, 1,4-phenylenedimethanamine, 3-phenyl-2-propen-1-ammonium, phenylbutylammonium, 4-tert-butylbenzylammonium, 3-(aminomethyl)piperidinium, and 4-(aminomethyl)piperidinium are preferred.

B in each of the general formulae [1] to [4] represents a metal atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Of those, lead, tin, and bismuth are preferred from the viewpoint of the overlap of electron orbits. Those metal atoms may be used alone or in combination thereof.

X in each of the general formulae [1] to [4] represents a halogen atom, and examples thereof include chlorine, bromine, and iodine. Those halogen atoms may be used alone or in combination thereof. Of those, a halogen atom is preferred because, when the halogen atom is incorporated into the structure, the above-mentioned crystal having a perovskite structure easily becomes soluble in an organic solvent, and hence the application to an inexpensive printing method or the like is enabled. Further, iodine is more preferred because the energy bandgap of the crystal having a perovskite structure narrows.

Specifically, as three-dimensional perovskite, two-dimensional perovskite, and mixed three-dimensional/two-dimensional perovskite, MAPbI₃, FAPbCl₃, FAPbI₃, MAPbIₓBr₃₋ₓ, MAPbIₓCl₃₋ₓ, Cs_{0.1}(MA_{0.17}FA_{0.83})_{0.9}Pb(I_{0.9}Br_{0.1})₃, Cs_{0.05}(MA_{0.17}FA_{0.83})_{0.95}Pb(I_{0.83}Br_{0.17})₃, {Csₓ₁(FAₓ₂MA₁₋ₓ₂)₁₋ₓ₁}ₓ₃Pb(Ix4Br₁₋ₓ₄)ₓ₅, Cs_{0.05}FA_{0.88}MA_{0.07}PbI_{2.56}Br_{0.44}, (FAPbI₃)_{0.95}(MAPbBr₃)_{0.05}, (FAPbI₃)_{0.85}(MAPbBr₃)_{0.15}, CsPbI₃, CsPbBr₃, Csₓ(MA)₁₋ₓPbI₃, Csₓ(FA)₁₋ₓPbI₃, MAₓ(FA)₁₋ₓPbI₃, MA_{0.17}FA_{0.83}Pb(I_{0.83}Br_{0.17})₃, Cs_{0.15}FA_{0.85}PbI_{2.55}Br_{0.45}, Cs_{0.05}FA_{0.88}MA_{0.07}PbI_{2.56}Br_{0.44}, Cs_{0.15}FA_{0.85}PbI_{2.55}Br_{0.45}, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (4FPEA)₂(FA_{0.3}MA_{0.7})₄Pb₅I₁₆, (PDMA)FA₂Pb₃I₁₀, (3AMPY)(MA)₃Pb₄I₁₃, (PDMA)MA₅Pb₆I₁₉, (PDMA)MA₃Pb₄I₁₃, (TTDMA)MA₃Pb₄I₁₃, (TTDMA)MA₄Pb₅I_{16,} (BA_{0.9}PEA_{0.1})₂MA₄Pb₅I₁₆, (BA_{0.9}PEA_{0.1})₂MA₃Pb₄I₁₃, (4FPEA)₂MA₃Pb₄I₁₃, (4FPEA)₂MA₄Pb₅I₁₆, (BA)₂MA₂Pb₃I₁₀, (BA)₂MA₃Pb₄I₁₃, (TEA)₂MA₂Pb₃I₁₀, (BA)₂MA₄Pb₅I₁₆, and (BA)₂MA₃Pb₄I₁₃ are preferred.

The A site, B site, or X site of each of the general formulae may be adjusted to be deficient or excessive in accordance with purposes, and the combinations of x1 to x5 may be changed in accordance with purposes. Particularly preferred ranges are 0.03≤x1≤0.10, 0.80≤x2≤0.96, 0.95≤x3≤1.05, 0.80≤x4≤0.96, and 2.95≤x5≤3.05. MACl may be included as a material for forming a perovskite crystal.

### [Table 1]

**Table 1**

| x1 | x2 | 1-x2 | x3 | x4 | 1-x4 | x5 |
|---|---|---|---|---|---|---|
| 0.05 | 0.83 | 0.17 | 1.00 | 0.83 | 0.17 | 3.00 |
| 0.05 | 0.83 | 0.17 | 0.99 | 0.83 | 0.17 | 2.99 |
| 0.05 | 0.83 | 0.17 | 0.98 | 0.83 | 0.17 | 2.98 |
| 0.05 | 0.83 | 0.17 | 0.97 | 0.83 | 0.17 | 2.97 |
| 0.05 | 0.83 | 0.17 | 0.96 | 0.83 | 0.17 | 2.96 |
| 0.05 | 0.83 | 0.17 | 1.01 | 0.83 | 0.17 | 3.01 |
| 0.05 | 0.83 | 0.17 | 1.02 | 0.83 | 0.17 | 3.02 |
| 0.05 | 0.83 | 0.17 | 1.03 | 0.83 | 0.17 | 3.03 |
| 0.05 | 0.83 | 0.17 | 1.04 | 0.83 | 0.17 | 3.04 |
| 0.05 | 0.83 | 0.17 | 1.00 | 0.95 | 0.05 | 3.00 |
| 0.05 | 0.83 | 0.17 | 0.97 | 0.95 | 0.05 | 2.97 |
| 0.05 | 0.83 | 0.17 | 0.98 | 0.95 | 0.05 | 2.98 |
| 0.05 | 0.83 | 0.17 | 0.99 | 0.95 | 0.05 | 2.99 |
| 0.05 | 0.83 | 0.17 | 1.01 | 0.95 | 0.05 | 3.01 |
| 0.05 | 0.83 | 0.17 | 1.02 | 0.95 | 0.05 | 3.02 |
| 0.05 | 0.83 | 0.17 | 1.03 | 0.95 | 0.05 | 3.03 |

In the above-mentioned specific examples, "MA" represents methylammonium, "FA" represents formamidinium, "PEA" represents phenethylammonium, "PTA" represents phenyltriethylammonium, "ThMA" represents 2-thiophenemethylammonium, "3BBA" represents 3-bromobenzylammonium, "3AMPY" represents 3-(aminomethyl)pyridine, "PDMA" represents 1,4-phenylenedimethanammonium, "TTDMA" represents thieno[3,2-b]thiophene-2,5-diyldimethanammonium, "4FPEA" represents 4-fluorophenethylammonium, "BA" represents butylammonium, and "TEA" represents 2-thiophenethylammonium.

The above-mentioned crystal having a perovskite structure preferably has a cubic structure in which the metal atom B, the organic molecules A, and the halogen atom X are arranged on a body-centered position, the respective corners, and a face-centered position, respectively. The details are not clear, but it is assumed that, when such structure is present, the orientation of an octahedron in a crystal lattice can be easily changed, and hence the mobility of an electron in the crystal having a perovskite structure increases, and the photoelectric conversion efficiency of the photoelectric conversion element is improved.

An organic-inorganic perovskite compound to be used in the present invention is preferably a crystalline semiconductor. The term "crystalline semiconductor" means a semiconductor that enables the measurement of an X-ray scattering intensity distribution to detect a scattering peak. When the organic-inorganic perovskite compound is the crystalline semiconductor, the mobility of the electron in the organic-inorganic perovskite compound increases, and the photoelectric conversion efficiency of the photoelectric conversion element is improved.

The thickness of the photoelectric conversion layer according to the present invention is preferably 5 to 2,000 nm. When the thickness is 5 nm or more, light can be sufficiently absorbed, and when the thickness is 2,000 nm or less, the generated charge can be transported to the respective electrodes. A more preferred lower limit is 50 nm or more, a more preferred upper limit is 1,200 nm, a still more preferred lower limit is 100 nm, and a still more preferred upper limit is 1,000 nm.

### [Charge-transporting Layer]

The photoelectric conversion element of the present invention includes the charge-transporting layer between the photoelectric conversion layer and the first electrode. In the photoelectric conversion element of the present invention, the charge-transporting layer contains a P-type semiconductor crystal serving as a charge-transporting substance, and a resin. In the photoelectric conversion element of the present invention, the P-type semiconductor crystal has a Lewis basic functional group. Further, the P-type semiconductor crystal has a functional group that may serve as a hydrogen bond donor. Still further, the P-type semiconductor crystal has at least one functional group selected from the group consisting of: a hydroxy group; a carboxy group; an amino group; an imino group; and a sulfo group. Those functional groups may be recognized by, for example, X-ray photoelectron spectroscopy (XPS) or nuclear magnetic resonance (NMR).

In the photoelectric conversion element of the present invention, it is preferred that the charge-transporting layer be in contact with the photoelectric conversion layer. The charge-transporting layer not only traps ions that have migrated from the photoelectric conversion layer but also directly interacts with defects on the perovskite surface, which is the photoelectric conversion layer, to thereby enhance a preventing effect on recombination of carriers.

In the photoelectric conversion element of the present invention, it is preferred that the P-type semiconductor crystal be dispersed with the resin. The charge-transporting material and the resin are easily and uniformly brought into contact with each other, and an effective charge distribution can be formed.

From the viewpoint of retention of a charge-transporting property, in the photoelectric conversion element of the present invention, the content of the P-type semiconductor crystal is preferably 5 to 30 times in terms of mass ratio with respect to the content of the resin in the charge-transporting layer. The content of the P-type semiconductor crystal and the content of the resin may be determined by, for example, X-ray photoelectron spectroscopy (XPS) or energy dispersive spectroscopy (EDS).

From the viewpoint of durability of the photoelectric conversion element, in the photoelectric conversion element of the present invention, the thickness of the charge-transporting layer is preferably 10 to 400 nm, more preferably 100 to 200 nm. The thickness of the charge-transporting layer may be determined by, for example, scanning electron microscope (SEM) observation or transmission electron microscope (TEM) observation of a cross section of the photoelectric conversion element.

In the photoelectric conversion element of the present invention, the resin is preferably a polyvinyl acetal resin, more preferably a polyvinyl butyral resin. It is presumed that the resin is easily brought into close contact with the charge-transporting material and that the functional groups of the resin interact with ions for forming the perovskite. In the present invention, chemical substances may be recognized by, for example, nuclear magnetic resonance (NMR).

From the viewpoint of a charge-transporting property, in the photoelectric conversion element of the present invention, the P-type semiconductor crystal is preferably a cyclic conjugated compound in which a plurality of pyrrole rings are bonded by conjugated bonds. In the present invention, the chemical substances may be recognized by, for example, nuclear magnetic resonance (NMR).

The charge-transporting layer may be formed by preparing a coating liquid for a charge-transporting layer containing the above-mentioned respective materials and a solvent, forming a coating film of the coating liquid on the photoelectric conversion layer, and drying the coating film. Examples of the solvent to be used for the coating liquid include an alcohol-based solvent, a ketone-based solvent, an ether-based solvent, a thioether-based solvent, an ester-based solvent, and an aromatic hydrocarbon-based solvent. Of those solvents, an alcohol-based solvent or an aromatic hydrocarbon-based solvent is preferred.

### [Second Charge-transporting Layer]

The photoelectric conversion element of the present invention may include a second charge-transporting layer between the first electrode and the charge-transporting layer. When the photoelectric conversion element includes the second charge-transporting layer, the transfer of carriers to an electrode may be facilitated.

A material for the second charge-transporting layer is not particularly limited, and examples thereof include a spirofluorene compound, a triphenylamine compound, a chrysene compound, a pyrene compound, a phthalocyanine compound, a carbazole compound, a fluorene compound, a phenylcyclohexane compound, a benzidine compound, a phenoxazine compound, a phenylenediamine compound, and a thiocyanate compound. The compound preferably has an aromatic ring from the viewpoint of the compatibility of a film interface, and Spiro-OMeTAD, PTAA, or a phthalocyanine compound is particularly preferred.

### [Electron-transporting Layer]

In the photoelectric conversion element of the present invention, the electron-transporting layer 4 may be arranged between the second electrode 3 and the photoelectric conversion layer 5 as illustrated in Fig. 1.

A material for the electron-transporting layer 4 is not particularly limited, and examples thereof include an N-type conductive polymer, an N-type low-molecular-weight organic semiconductor, an N-type metal oxide, an N-type metal sulfide, a halogenated alkali metal, an alkali metal, and a surfactant. Specific examples thereof include a cyano group-containing polyphenylene vinylene, a boron-containing polymer, bathocuproine, bathophenanthrene, hydroxyquinolinatoaluminum, an oxadiazole compound, a benzimidazole compound, a naphthalenetetracarboxylic acid compound, a perylene derivative, a phosphine oxide compound, a phosphine sulfide compound, a fluoro group-containing phthalocyanine, titanium oxide, zinc oxide, indium oxide, tin oxide, gallium oxide, tin sulfide, indium sulfide, and zinc sulfide.

A preferred lower limit of the thickness of the electron-transporting layer 4 is 1 nm, and a preferred upper limit thereof is 2,000 nm. When such thickness is 1 nm or more, a hole can be sufficiently blocked, and when the thickness is 2,000 nm or less, the electron-transporting layer 4 is less liable to serve as a resistance at the time of the electron transportation, and hence the photoelectric conversion efficiency is improved. A more preferred lower limit of the thickness is 3 nm, a more preferred upper limit thereof is 1,000 nm, a still more preferred lower limit thereof is 5 nm, and a still more preferred upper limit thereof is 500 nm.

### <Application Examples>

Application examples of the present invention are directed to a photoelectric conversion apparatus, a moving body, and a building material.

### [Photoelectric Conversion Apparatus]

A photoelectric conversion apparatus of the present invention includes the above-mentioned photoelectric conversion element. The photoelectric conversion apparatus may be formed by using the plurality of photoelectric conversion elements of the present invention. When the plurality of photoelectric conversion elements are connected, such photoelectric conversion apparatus may also be referred to as "photoelectric conversion cell" or "photoelectric conversion module." Photoelectric conversion elements having different absorption wavelengths may be laminated as the photoelectric conversion elements to increase an output voltage. In addition, the photoelectric conversion apparatus includes the photoelectric conversion element of the present invention and an inverter. The inverter may be a converter for converting a DC voltage to an AC voltage. The photoelectric conversion apparatus may include an electricity storage unit connected to the photoelectric conversion element. The electricity storage unit is not limited as long as the electricity storage unit can store electricity. Examples thereof include a secondary battery using lithium ions, an all-solid-state battery, and an electric double layer capacitor.

### [Moving Body]

A moving body of the present invention includes the above-mentioned photoelectric conversion element. Fig. 2 is a perspective view for schematically illustrating a moving body including the photoelectric conversion element according to one embodiment of the present invention. A moving body 30 includes a photoelectric conversion element 31 of the present invention and a body 32 including the photoelectric conversion element 31. The photoelectric conversion element 31 is arranged on the position of the body 32 at which ambient light can be received. When the moving body 30 is an automobile, the photoelectric conversion element 31 may be arranged on a roof. Electric energy obtained by the photoelectric conversion element 31 may serve as the power of the moving body 30 or the power of any other electric equipment. Electric energy generated from the power of the moving body 30 may be used for the power of the photoelectric conversion element 31. When the moving body 30 is an automobile, friction energy generated with a brake may be converted into electric energy to be used for the control of the photoelectric conversion element 31.

The moving body 30 may be, for example, an automobile, a ship, an airplane, or a drone. The configuration of the body 32 of the moving body 30 is not particularly limited, but is preferably formed of a material having high strength.

### [Building Material]

A building material of the present invention includes the above-mentioned photoelectric conversion element. Fig. 3 is a perspective view for schematically illustrating a building material including the photoelectric conversion element according to one embodiment of the present invention. A building material 40 may be a roof of a building. The building material 40 of this embodiment includes a photoelectric conversion element 41 of the present invention, a protective member 42 for protecting the photoelectric conversion element 41, a heat dissipation member 43, and exteriors 44a and 44b.

The building material 40 of the present invention may include the heat dissipation member 43 having a thermal conductivity higher than that of the photoelectric conversion element 41. When the building material 40 is used for a roof or the like, the temperature of the photoelectric conversion element 41 may be increased by sunlight, and hence the photoelectric conversion efficiency may be reduced. The reduction of the photoelectric conversion efficiency can be suppressed by using the heat dissipation member 43. Examples of the heat dissipation member 43 include a metal, an alloy, a liquid metal, and a liquid resin.

In addition, the building material 40 of the present invention may include the exteriors 44a and 44b. The exterior 44a and the exterior 44b may show different colors, or may show the same color. The exterior 44a and the exterior 44b may be formed of the same member, or may be formed of different members. A paint or a transparent substrate may be used as each of the exteriors. An exterior having small light absorption and a high heat-shielding property is preferred.

### [Examples]

The present invention is described in more detail below by way of Examples and Comparative Examples. The present invention is by no means limited to the following Examples without departing from the gist thereof. In the description of the following Examples, the term "part(s)" is by mass unless otherwise specified.

### <Production of P-type Semiconductor Crystal Particle 1>

### Step (1)

Under a nitrogen flow atmosphere, 5.46 parts of orthophthalonitrile and 45 parts of α-chloronaphthalene were loaded into a reaction kettle. After that, the mixture was heated so that its temperature was increased to 30°C, followed by the maintenance of the temperature. Next, 3.75 parts of gallium trichloride was loaded into the mixture at the temperature (30°C). The moisture concentration of the mixed liquid at the time of the loading was 150 ppm.

After that, the temperature of the mixed liquid was increased to 200°C. Next, under a nitrogen flow atmosphere, the mixed liquid was subjected to a reaction at a temperature of 200°C for 4.5 hours, and was then cooled. The product was filtered when its temperature reached 150°C. The resultant filter residue was subjected to dispersion washing with N,N-dimethylformamide at a temperature of 140°C for 2 hours, and was then filtered. The resultant filter residue was washed with methanol, and was then dried to provide a chlorogallium phthalocyanine particle in a yield of 71%.

### Step (2)

4.65 Parts of the chlorogallium phthalocyanine particle was dissolved in 139.5 parts of concentrated sulfuric acid at a temperature of 10°C, and the solution was dropped into 620 parts of ice water under stirring so that the particle was reprecipitated, followed by filtration with a filter press under reduced pressure. At this time, No. 5C (manufactured by Advantec Toyo Kaisha, Ltd.) was used as a filter.

The resultant wet cake (filter residue) was subjected to dispersion washing with 2% ammonia water for 30 minutes, and was then filtered with the filter press. Next, the resultant wet cake (filter residue) was subjected to dispersion washing with ion-exchanged water, and then its filtration with the filter press was repeated three times.

Finally, the filter residue was freeze-dried to provide a hydroxygallium phthalocyanine particle (hydrous hydroxygallium phthalocyanine particle) having a solid content of 23 mass% in a yield of 71%. The hydroxygallium phthalocyanine particle was dried with a hyper-dry dryer (product name: HD-06R, frequency (oscillatory frequency): 2,455 MHz±15 MHz, manufactured by Biocon (Japan) Ltd.). Thus, a hydroxygallium phthalocyanine (OHGaPc) particle (crystal) having a water content of 1.0 mass% or less was obtained.

### Step (3)

5 Parts of the hydroxygallium phthalocyanine particle was mixed with 5 parts of an N-methylformamide solvent, and the mixture was subjected to dispersion treatment for 6 hours with a sand mill (TSG-1/4G-4U, manufactured by Igarashi Machine Production Co., Ltd. (currently AIMEX Co., Ltd.), disc diameter: 70 mm, number of discs: 5) containing 5 parts of glass beads, followed by filtration and drying to provide a P-type semiconductor crystal particle 1 (specific gravity: 1.6).

### <Production of Resin Solution 1>

1.0 Parts of a polyvinyl butyral resin (product name: S-LEC (trademark) BM-2, manufactured by Sekisui Chemical Co., Ltd., specific gravity: 1.6) was dissolved in 19 parts of 2-propanol by stirring for 24 hours to provide a resin solution 1.

### (Example 1)

### [Formation of Electron-transporting Layer]

A square glass substrate with ITO having a side of 25 mm was washed, and a solution obtained by diluting a tin(II) oxide colloidal dispersion with water to a volume ratio of 1/7 was applied onto the glass substrate by spin coating at 5,000 rpm for 30 seconds. After that, the resultant was heated at 150°C for 30 minutes, and was dried under a humidity environment with a dew point temperature of -20°C to form an electron-transporting layer.

### [Formation of Photoelectric Conversion Layer]

Lead iodide (1.2 M), lead bromide (0.15 M), formamidinium iodide (1.0 M), methylammonium bromide (0.15 M), and cesium iodide (0.13 M) were dissolved in a mixed solvent containing N,N-dimethylformamide and dimethyl sulfoxide at a volume ratio of 4:1 to prepare a photoelectric conversion layer coating liquid. The coating liquid was applied onto the electron-transporting layer by spin coating at 2,700 rpm for 10 seconds and then at 5,000 rpm for 20 seconds. Five seconds before the end of the spin coating, 150 µL of chlorobenzene was dropped onto the substrate. Finally, the substrate was heated at 130°C for 15 minutes to form a photoelectric conversion layer formed of Cs_{0.1}(MA_{0.17}FA_{0.83})_{0.9}Pb(I_{0.9}Br_{0.1})₃.

### [Formation of Charge-transporting Layer]

0.1 Parts of the P-type semiconductor crystal particle 1 and 0.01 parts of a calixarene compound (see Japanese Patent Laid-Open No. 2003-207913) were mixed with 10.6 parts of 2-propanol, and 11 parts of beads (zirconia beads, Torayceram (trademark) zirconia beads, 0.3 mm) were loaded into the mixture, followed by dispersion with a paint shaker (manufactured by Toyo Seiki Co., Ltd.) for 3 hours. After that, 0.2 parts of the resin solution 1 was added thereto, and dispersion with the paint shaker was performed again for 4 hours to prepare a charge-transporting layer solution. The charge-transporting layer solution was applied onto the photoelectric conversion layer by spin coating at 1,000 rpm for 30 seconds, and the resultant was heated at 60°C for 10 minutes to form a charge-transporting layer having a thickness of about 150 nm.

### [Introduction of Second Charge-transporting Layer]

0.08 Parts of Spiro-OMeTAD serving as a material for a second charge-transporting layer was dissolved in 1.13 parts of chlorobenzene. The chlorobenzene solution was mixed with 0.019 parts of an acetonitrile solution (2 M) of lithium bis(trifluoromethanesulfonyl)imide, 0.030 parts of 4-tert-butylpyridine (TBP), and 0.016 parts of an acetonitrile solution (0.25 M) of [tris(2-(1H-pyrazol-1-yl)-4-tert-butylpyridine)cobalt(III)
tris(bis(trifluoromethylsulfonyl)imide)] to prepare a material solution for a second charge-transporting layer. The material solution was applied onto the charge-transporting layer by a spin coating method at 2,000 rpm for 30 seconds to form a second charge-transporting layer.

### [Formation of First Electrode]

Ten gold electrodes each having a thickness of 70 nm and an area of 0.09 cm² were formed on the second charge-transporting layer by a vacuum vapor deposition method. Thus, a photoelectric conversion element was obtained.

### [Analysis of Amount of Compound]

The electrode surface of the photoelectric conversion element was peeled off so that the surface of the charge-transporting layer was exposed. The surface of the charge-transporting layer was wiped with a cotton swab or the like with a solvent, dissolved in deuterated sulfuric acid, and subjected to ¹H-NMR measurement (apparatus: AVANCE III 500, manufactured by BRUKER). In addition, the presence of a compound was determined by subjecting the peeled-off charge-transporting layer components to mass and structure analyses through GPC and MALDI-TOF-MS, IR, gas chromatography, and elemental analysis, such as XPS or EDX.

In addition, the thickness of the layer was determined with a cross-sectional SEM (apparatus: SmartSEM, manufactured by Carl Zeiss Co., Ltd.) after the cutting of the photoelectric conversion element and the fixing of the sample to a tilted sample stage.

### (Example 2)

A photoelectric conversion element is obtained in the same manner as in Example 1 except that the thickness of the charge-transporting layer is changed to 200 nm.

### (Example 3)

A photoelectric conversion element is obtained in the same manner as in Example 1 except that the mass ratio of the P-type semiconductor crystal particle 1 with respect to the resin is changed to 3 times.

### (Example 4)

A photoelectric conversion element is obtained in the same manner as in Example 1 except that the mass ratio of the P-type semiconductor crystal particle 1 with respect to the resin is changed to 5 times.

### (Example 5)

A photoelectric conversion element is obtained in the same manner as in Example 1 except that the mass ratio of the P-type semiconductor crystal particle 1 with respect to the resin is changed to 30 times.

### (Example 6)

A photoelectric conversion element is obtained in the same manner as in Example 1 except that the mass ratio of the P-type semiconductor crystal particle 1 with respect to the resin is changed to 35 times.

### (Example 7)

A photoelectric conversion element is obtained in the same manner as in Example 1 except that the thickness of the charge-transporting layer is changed to 89 nm.

### (Example 8)

A photoelectric conversion element is obtained in the same manner as in Example 1 except that the thickness of the charge-transporting layer is changed to 300 nm.

### (Example 9)

A photoelectric conversion element is obtained in the same manner as in Example 1 except that the P-type semiconductor crystal particle 1 is changed to tetraphenylporphyrin (TPP). The tetraphenylporphyrin (TPP) to be used as the P-type semiconductor crystal particle 1 is crystalline.

### (Example 10)

A photoelectric conversion element is obtained in the same manner as in Example 1 except that the P-type semiconductor crystal particle 1 is changed to 4,4',4",4‴-(porphine-5,10,15,20-tetrayl)tetrakis(benzoic acid). The 4,4',4",4‴-(porphine-5,10,15,20-tetrayl)tetrakis(benzoic acid) to be used as the P-type semiconductor crystal particle 1 is crystalline.

### (Example 11)

A photoelectric conversion element is obtained in the same manner as in Example 1 except that the P-type semiconductor crystal particle 1 is changed to 1-aminoanthraquinone-2-sulfonic acid. The 1-aminoanthraquinone-2-sulfonic acid to be used as the P-type semiconductor crystal particle 1 is crystalline.

### (Example 12)

A photoelectric conversion element is obtained in the same manner as in Example 1 except that the calixarene compound is not used.

### (Example 13)

A photoelectric conversion element is obtained in the same manner as in Example 1 except that the second charge-transporting layer is not arranged.

### (Example 14)

A photoelectric conversion element is obtained in the same manner as in Example 1 except that the resin is changed to poly(3-hexylthiophene-2,5-diyl) (P3HT).

### (Example 15)

A photoelectric conversion element is obtained in the same manner as in Example 1 except that the P-type semiconductor crystal particle 1 is changed to a quinacridone particle. The quinacridone particle to be used as the P-type semiconductor crystal particle 1 is crystalline.

### (Comparative Example 1)

In the production process for the P-type semiconductor crystal particle 1, the step (3) was not performed, and a P-type semiconductor particle 2 that was not converted into a crystal was obtained. A photoelectric conversion element was obtained in the same manner as in Example 1 except that the P-type semiconductor particle 2 was used instead of the P-type semiconductor crystal particle 1 in the formation of the charge-transporting layer. The P-type semiconductor particle 2 that was not converted into a crystal is amorphous.

### (Comparative Example 2)

A photoelectric conversion element is obtained in the same manner as in Example 1 except that the P-type semiconductor crystal particle 1 is changed to a copper phthalocyanine particle.

### (Comparative Example 3)

A photoelectric conversion element is obtained in the same manner as in Example 1 except that the resin is not used.

### (Comparative Example 4)

A photoelectric conversion element is obtained in the same manner as in Example 1 except that the P-type semiconductor crystal particle 1 is changed to Spiro-OMeTAD and the polyvinyl butyral is changed to polymethyl methacrylate (PMMA, manufactured by Sigma-Aldrich LLC).

### [Evaluation]

A power supply (236 model, manufactured by Keithley Instruments) was connected between the electrodes of the photoelectric conversion element produced in Example 1, and its photoelectric conversion efficiency was measured by: irradiating the element with constant light through use of a solar simulator (manufactured by Yamashita Denso Corporation) having an intensity of 110 mW/cm²; and measuring the generated current and voltage. In addition, each of the ten electrodes for each element was subjected to the measurement, and the maximum of the measured values was adopted as the representative value of the element. After that, light of 10,000 Lx was continuously applied to the element with a white LED, and its photoelectric conversion efficiency after 60 days was measured. Then, the maintenance rate of the photoelectric conversion efficiency after 60 days with respect to the resultant initial photoelectric conversion efficiency was evaluated as the evaluation of durability. Those results together with the initial photoelectric conversion efficiency are shown in Table 2.

The photoelectric conversion elements of Examples 2 to 15 and Comparative Examples 1 to 4 are each evaluated for its initial photoelectric conversion efficiency and its maintenance rate of the photoelectric conversion efficiency after 60 days in the same manner as in Example 1. The results are shown in Table 2.

### [Table 2]

**Table 2**

| | Charge-transporting layer | | Resin | Content ratio (mass ratio) of P-type semiconductor crystal particle with respect to content of resin | Dispersant | Second charge-transporting layer | Photoelectric conversion efficiency [%] | Maintenance rate of photoelectric conversion efficiency [%] |
|---|---|---|---|---|---|---|---|---|
| | P-type semiconductor crystal particle | Thickness [nm] | Name | P/B | | | | |
| Example 1 | Hydroxygallium phthalocyanine (with crystal conversion step (3)) | 150 | BM-2 | 10 | Present | Present | 21.0 | 95.2 |
| Example 2 | Hydroxygallium phthalocyanine (with crystal conversion step (3)) | 200 | BM-2 | 10 | Present | Present | 20.6 | 95.3 |
| Example 3 | Hydroxygallium phthalocyanine (with crystal conversion step (3)) | 150 | BM-2 | 3 | Present | Present | 16.2 | 97 |
| Example 4 | Hydroxygallium phthalocyanine (with crystal conversion step (3)) | 150 | BM-2 | 5 | Present | Present | 17.3 | 96.1 |
| Example 5 | Hydroxygallium phthalocyanine (with crystal conversion step (3)) | 150 | BM-2 | 30 | Present | Present | 19.1 | 78.6 |
| Example 6 | Hydroxygallium phthalocyanine (with crystal conversion step (3)) | 150 | BM-2 | 35 | Present | Present | 17.0 | 85.5 |
| Example 7 | Hydroxygallium phthalocyanine (with crystal conversion step (3)) | 89 | BM-2 | 10 | Present | Present | 19.5 | 90.7 |
| Example 8 | Hydroxygallium phthalocyanine (with crystal conversion step (3)) | 300 | BM-2 | 10 | Present | Present | 18.3 | 96.8 |
| Example 9 | TPP | 150 | BM-2 | 10 | Present | Present | 15.9 | 93 |
| Example 10 | 4,4',4",4‴-(Porphine-5,10,15,20-tetrayl)tetrakis(benzoic acid) | 150 | BM-2 | 10 | Present | Present | 17.1 | 82.1 |
| Example 11 | 1-Aminoanthraquinone-2-sulfonic acid | 150 | BM-2 | 10 | Present | Present | 16.2 | 84.5 |
| Example 12 | Hydroxygallium phthalocyanine (with crystal conversion step (3)) | 150 | BM-2 | 10 | Absent | Present | 16.5 | 87.2 |
| Example 13 | Hydroxygallium phthalocyanine (with crystal conversion step (3)) | 150 | BM-2 | 10 | Present | Absent | 18.0 | 86.6 |
| Example 14 | Hydroxygallium phthalocyanine (with crystal conversion step (3)) | 150 | P3HT | 10 | Present | Present | 18.2 | 93.8 |
| Example 15 | Quinacridone | 150 | BM-2 | 10 | Present | Present | 15.3 | 83.3 |
| Comparative Example 1 | Hydroxygallium phthalocyanine (without crystal conversion step (3)) | 150 | BM-2 | 10 | Present | Present | 16.1 | 94.4 |
| Comparative Example 2 | Copper phthalocyanine | 150 | BM-2 | 10 | Present | Present | 12.8 | 73.9 |
| Comparative Example 3 | Hydroxygallium phthalocyanine (with crystal conversion step (3)) | 150 | × | 10 | Present | Present | 13.9 | 72.2 |
| Comparative Example 4 | Spiro-OMeTAD | 150 | PMM A | 10 | Absent | Present | 12.6 | 70.6 |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-184761 filed on October 27, 2023, Japanese Patent Application No. 2023-184756 filed on October 27, 2023, Japanese Patent Application No. 2023-184750 filed on October 27, 2023, Japanese Patent Application No. 2023-216294 filed on December 21, 2023, Japanese Patent Application No. 2023-216296 filed on December 21, 2023, Japanese Patent Application No. 2023-216299 filed on December 21, 2023, Japanese Patent Application No. 2024-022244 filed on February 16, 2024, Japanese Patent Application No. 2024-022251 filed on February 16, 2024, Japanese Patent Application No. 2024-022246 filed on February 16, 2024, Japanese Patent Application No. 2024-077353 filed on May 10, 2024, and Japanese Patent Application No. 2024-186698 filed on October 23, 2024, and the entire contents thereof are incorporated herein by reference.

### [Reference Signs List]

1 photoelectric conversion element
2 substrate
3 second electrode
4 electron-transporting layer
5 photoelectric conversion layer
6 charge-transporting layer
7 first electrode
8 second charge-transporting layer
30 moving body
31, 41 photoelectric conversion element
32 body
40 building material
42 protective member
43 heat dissipation member
44a, 44b exterior

## Claims

1. A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element comprises a charge-transporting layer between the photoelectric conversion layer and the first electrode,
wherein the charge-transporting layer contains a P-type semiconductor crystal and a resin, and
wherein the P-type semiconductor crystal has at least one functional group selected from the group consisting of: a hydroxy group; a carboxy group; an amino group; an imino group; and a sulfo group.

2. A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element comprises a charge-transporting layer between the photoelectric conversion layer and the first electrode,
wherein the charge-transporting layer contains a P-type semiconductor crystal and a resin, and
wherein the P-type semiconductor crystal has a functional group that may serve as a hydrogen bond donor.

3. A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element comprises a charge-transporting layer between the photoelectric conversion layer and the first electrode,
wherein the charge-transporting layer contains a P-type semiconductor crystal and a resin, and
wherein the P-type semiconductor crystal has a Lewis basic functional group.

4. The photoelectric conversion element according to any one of claims 1 to 3, wherein the charge-transporting layer is in contact with the photoelectric conversion layer.

5. The photoelectric conversion element according to any one of claims 1 to 4, wherein, in the charge-transporting layer, the P-type semiconductor crystal is dispersed with the resin.

6. The photoelectric conversion element according to any one of claims 1 to 5, wherein a content of the P-type semiconductor crystal in the charge-transporting layer is 5 to 30 times in terms of mass ratio with respect to a content of the resin in the charge-transporting layer.

7. The photoelectric conversion element according to any one of claims 1 to 6, wherein a thickness of the charge-transporting layer is 10 to 400 nm.

8. The photoelectric conversion element according to any one of claims 1 to 6, wherein a thickness of the charge-transporting layer is 100 to 200 nm.

9. The photoelectric conversion element according to any one of claims 1 to 8, wherein the photoelectric conversion element comprises a second charge-transporting layer between the first electrode and the charge-transporting layer.

10. The photoelectric conversion element according to any one of claims 1 to 9, wherein the resin is a polyvinyl acetal resin.

11. The photoelectric conversion element according to any one of claims 1 to 10, wherein the P-type semiconductor crystal is a cyclic conjugated compound in which a plurality of pyrrole rings are bonded by conjugated bonds.

12. A photoelectric conversion apparatus comprising the photoelectric conversion element of any one of claims 1 to 11.

13. A moving body comprising the photoelectric conversion element of any one of claims 1 to 11.

14. A building material comprising the photoelectric conversion element of any one of claims 1 to 11.
